# EUROPÄISCHE PATENTSCHRIFT

(11) **EP 2 903 662 B1**
(45) Veröffentlichungstag und Bekanntmachung des Hinweises auf die Patenterteilung: **24.10.2018**
(21) Anmeldenummer: 13780052.0
(22) Anmeldetag: 17.09.2013
(51) Int. Cl.: A61M 5/168, A61M 5/24

(54) **ABGABEVORRICHTUNG FUER MEDIKAMENTE**
ADMINISTRATION DEVICE FOR DRUGS
DISPOSITIF D'ADMINISTRATION DE MEDICAMENTS

(30) Priorität: 05.10.2012 AT 504282012
(43) Veröffentlichungstag der Anmeldung: 12.08.2015
(73) Patentinhaber: Seibersdorf Labor GmbH, 2444 Seibersdorf (AT); AIT Austrian Institute of Technology GmbH, 1220 Wien (AT)
(72) Erfinder: BAMMER, Manfred, A-1220 Wien (AT); SCHMID, Gernot, A-2833 Bromberg (AT); PUTZ, Otmar, A-2833 Bromberg (AT)
(74) Vertreter: Wildhack & Jellinek
(86) Internationale Anmeldenummer: PCT/AT2013/050187
(87) Internationale Veröffentlichungsnummer: WO 2014/052997

(56) Entgegenhaltungen:
- WO-A2-2007/107558
- US-A- 4 237 878
- US-A- 4 749 988
- US-A- 6 110 148
- US-A1- 2003 064 147

## Beschreibung

Die Erfindung betrifft eine Abgabevorrichtung zur Abgabe von Flüssigkeiten, insbesondere von flüssigen Medikamenten an Personen, gemäß dem Oberbegriff des Patentanspruches 1. Weiters betrifft die Erfindung ein Verfahren zur Bestimmung und Validierung des Füllstandes in einem Behälter gemäß dem Oberbegriff des Patentanspruches 17.

Die Erfindung kann insbesondere im Gesundheitswesen, beispielsweise in der Medizintechnik, Pharma- und Biotechnologie, Medizin und Pflege, Studien, usw. zur Überwachung der Abgabe von Medikamenten an Patienten eingesetzt werden.

Aus dem Stand der Technik, wie etwa WO 2007/107558 A2, sind verschiedene Vorrichtungen zur Abgabe von Flüssigkeiten bekannt, bei denen die abgegebene Flüssigkeit kapazitiv bestimmt wird. Aufgabe der vorliegenden Erfindung ist es, Fehlfunktionen bei kapazitiven Füllstandsdetektionen wirksam zu erkennen und eine Invalidierung von kapazitiven Füllstands-Messergebnissen zu ermöglichen. Weiters ist es Aufgabe der Erfindung, möglichst gute und zuverlässige Resultate zu erhalten.

Die Erfindung löst diese Aufgabe bei einer Abgabevorrichtung der eingangs genannten Art mit dem kennzeichnenden Merkmal des Patentanspruches 1.

Zudem löst die Erfindung die Aufgabe bei einem Verfahren der eingangs genannten Art mit dem Merkmal des Kennzeichens des Patentanspruches 17.

Erfindungsgemäß sind bei einer Abgabevorrichtung zur Abgabe von Flüssigkeiten, insbesondere von flüssigen Medikamenten an Personen, vorgesehen:
- ein mit der Flüssigkeit gefüllten Behälter, der an einem Ende eine Öffnung zur Abgabe der Flüssigkeit aufweist, sowie
- zumindest ein Paar von im Außenbereich, insbesondere an der Wand, des Behälters einander gegenüberliegend angeordneten kapazitiven Messelektroden zur Bestimmung der Permittivität des jeweiligen Mediums im Zwischenbereich zwischen den Messelektroden, umfassend eine die Messelektroden mantelförmig umgebende und um den Behälter angeordnete Abschirmung.

Störungen, die durch das Berühren des Behälters während des Messvorganges aufgrund kapazitiver Effekte verursacht werden, werden mit der Erfindung wirksam vermieden.

Insbesondere kann durch die erfindungsgemäße Maßnahme vermieden werden, dass eine Berührung der Messelektroden durch die Hände eines Menschen oder eine Verfälschung des Feldes im Bereich der Messelektroden durch die Hände eines Menschen zu Änderungen des Füllstandsmesswertes führen.

Die Abschirmung ist als mit Leiterbahnen aus elektrisch leitfähigem Material beschichtete Folie ausgebildet. In einer bevorzugten Ausführungsform ist diese Folie um den Behälter angeordnet oder gewickelt. Eine solche Abschirmung verhindert die Verfälschung von Messergebnissen besonders vorteilhaft.

Vorteilhafterweise ist der Bereich zwischen der Flüssigkeit und den Messelektroden frei von der Abschirmung.

Um eine Verschlechterung der Messergebnisse durch Einflüsse der Abschirmung zu vermeiden, kann vorgesehen sein, dass die Abschirmung von den Messelektroden in radialer Richtung beabstandet ist.

Um eine verbesserte Abschirmung zu erreichen und gleichzeitig eine Befestigung eines Halbleiterchips und einer Antenne im Bereich der Abschirmung oder auf der Folie zu ermöglichen, kann vorgesehen sein, dass die Abschirmung als mit Leitern in Form von Leiterbahnen beschichtete, insbesondere um den Behälter gewickelte, Folie ausgebildet ist, wobei vorzugsweise auf der Folie eine Kapazitätsmesschaltung, eine Recheneinheit und ein Kommunikationscontroller, insbesondere in Form eines Halbleiterchips, sowie eine Antenne aufgebracht sind.

Um eine die Funkkommunikation mit der Antenne beeinträchtigende Veränderung des von einem externen Datenkommunikationsgerätes erzeugten elektromagnetischen Feldes zu vermeiden und gleichzeitig eine gute elektrische Abschirmung der Messelektroden zu ermöglichen, kann vorgesehen sein, dass die Leiter schleifenfrei und/oder frei von geschlossenen Leiterschleifen ausgebildet sind.

Die Ausgestaltung der Abschirmung, die gleichzeitig zur Berührungsdetektion verwendet werden kann und zudem eine Funkkommunikation mit einer auf die Abschirmung aufgebrachten Antenne ermöglicht, sieht vor, dass auf der Folie drei separate Leiter ausgebildet sind, wobei der erste und der zweite Leiter als ineinandergreifende Kammleiter ausgebildet sind und der dritte Leiter mäanderförmig ausgebildet zwischen den beiden Kammleitern liegt.

Eine bevorzugte Maßnahme zur Bestimmung des Füllstandes der Flüssigkeit im Inneren des Behälters sieht vor, dass die beiden gegenüberliegenden Messelektroden an eine Kapazitätsmesseinrichtung angeschlossen sind.

Zur einfachen Ermittlung des Füllstandes kann vorgesehen sein, dass der von der Kapazitätsmesseinrichtung ermittelte Kapazitätswert einer Recheneinheit zugeführt ist, die aufgrund des ermittelten Kapazitätswerts mittels einer vorgegebenen abgespeicherten Kalibrierfunktion den Füllstand der Flüssigkeit im Behälter bestimmt und an ihrem Ausgang zur Verfügung hält.

Eine besonders wirksame Abschirmung mit guter Abschirmungswirkung kann erzielt werden, indem einer der drei Leiter, insbesondere der als Kammleiter ausgebildete zweite Leiter, mit dem Massenanschluss der Kapazitätsmesseinrichtung verbunden ist.

Um Berührungen oder Verfälschungen der Kapazitätsmessung vorteilhaft feststellen zu können, kann ein außerhalb oder im Bereich der Abschirmung angeordneter, insbesondere kapazitiver, Berührungssensor vorgesehen sein.

Eine produktionstechnische einfach zu fertigende Variante sieht vor, dass der Berührungssensor den ersten Kammleiter und den mäanderförmigen Leiter der Abschirmung als Sensorelektroden umfasst.

Hierbei kann zur Detektion von Berührungen vorgesehen sein, dass die Sensorelektroden des Berührungssensors an eine weitere Kapazitätsmesseinrichtung angeschlossen sind, und dass vorzugsweise der von der weiteren Kapazitätsmesseinrichtung ermittelte weitere Kapazitätswert der Recheneinheit zugeführt ist, und die Recheneinheit für den Fall, dass der ermittelte weitere Kapazitätswert einen vorgegebenen Schwellenwert übersteigt, die Weiterleitung des von ihr ermittelten Füllstands unterdrückt oder als ungültig kennzeichnet.

Ein vorteilhafter Behälter zur Aufnahme von Flüssigkeiten, der einfach entleert werden kann und dessen Füllstand einfach festgestellt werden kann, sieht vor, dass der Behälter ein Innenvolumen aufweist, das abgesehen vom Bereich der Öffnung einen konstanten Innenquerschnitt aufweist,
wobei ein den Behälter und die darin befindliche Flüssigkeit abschließender und abdichtender Kolben vorgesehen ist, dessen Außenquerschnitt dem Querschnitt des Innenvolumens des Behälters entspricht und der im Inneren des Behälters verschiebbar angeordnet ist, sodass bei Vorschub des Kolbens zur Öffnung hin die Flüssigkeit durch die Öffnung aus dem Behälter abgegeben wird.

Zur genaueren Bestimmung des Füllstandes kann vorgesehen sein, dass am Behälter eine Vielzahl von Paaren von zusätzlichen Messelektroden angeordnet sind, wobei insbesondere für jedes Paar von zusätzlichen Messelektroden jeweils eine zusätzliche dem Paar von zusätzlichen Messelektroden nachgeschaltete Kapazitätsmesseinrichtung vorgesehen ist, die den ermittelten Kapazitätswert an die Recheneinheit abgibt.

Eine vorteilhafte Elektrodenanordnung, die eine genaue Füllstandsbestimmung ermöglicht, sieht vor, dass die jeweils einander paarweise zugeordneten Messelektroden einander in Umfangsrichtung des Behälters, insbesondere diametral, gegenüberliegen und insbesondere in Richtung des Vorschubs des Kolbens auf derselben Höhe liegen.

Hierbei kann zusätzlich zur Verbesserung der Detektionsgenauigkeit vorgesehen sein, dass jeweils benachbarte Paare von Messelektroden in Richtung des Vorschubs des Kolbens beabstandet angeordnet sind und/oder
dass die Breite der Messelektroden in Richtung des Vorschubs des Kolbens der Breite des Kolbens in dessen Vorschubrichtung entspricht.

Bevorzugte Ausführungen der Messelektroden mit einem einfachen Aufbau sehen vor,
- dass die Messelektroden flächenhaft auf der Außenoberfläche des Behälters angeordnet sind und insbesondere die Form eines Rechtecks, eines Dreiecks, eines Trapezes oder eines Parallelogramms aufweisen, und/oder
- dass je zwei der einander paarweise zugeordneten Messelektroden durch zwei ineinandergreifende Kammleiter ausgebildet sind, die im Außenbereich, insbesondere an der Außenwand, des Behälters angeordnet sind.

Um einen einfachen Austausch der Behälter zu ermöglichen, kann vorgesehen sein, dass außerhalb des Behälters zwischen dem Behälter und der Abschirmung ein Träger angeordnet ist, auf dem die Messelektroden angeordnet sind, wobei der Träger vorzugsweise am Behälter anliegt und/oder dass die Messelektroden auf der am Behälter anliegenden Wand des Trägers angeordnet sind.

Vorteilhafterweise kann der ermittelte Füllstand an ein externes Kommunikationsgerät übermittelt werden. Hierbei kann vorgesehen sein, dass an die Recheneinheit ein Kommunikationscontroller mit einer ihm nachgeschalteten Antenne angeschlossen ist. Vorteilhafterweise kann für eine platzsparendere Anordnung vorgesehen sein, dass die Antenne im Au ßenbereich der Abschirmung oder unmittelbar auf der Abschirmung, jedoch nicht elektrisch leitfähig mit dieser verbunden, angeordnet ist.

Weiters betrifft die Erfindung ein Verfahren zur Bestimmung und Validierung des Füllstandes in einem Behälter, der insbesondere in einer erfindungsgemäßen Abgabevorrichtung angeordnet ist, wobei zumindest ein Paar von im Außenbereich des Behälters einander gegenüberliegend angeordneten, insbesondere mit einer äußeren Abschirmung versehenen, Messelektroden zur Kapazitätsmessung vorgesehen ist, wobei die Kapazität zwischen den beiden Messelektroden ermittelt wird und aufgrund der ermittelten Kapazität gemäß einer vorgegebenen Kalibrierfunktion ein Füllstandswert bestimmt wird,
Erfindungsgemäß ist bei einem solchen Verfahren vorgesehen,
- dass eine weitere Kapazität mit im Außenbereich der Messelektroden im Bereich der Abschirmung, insbesondere auf der Abschirmung, angeordneten Leitern ermittelt wird,
- dass die weitere Kapazität mit einem Schwellenwert verglichen wird, und
- dass der Füllstandswert nur dann als gültig angesehen wird, wenn die weitere Kapazität unterhalb des Schwellenwerts liegt.

Mit einem solchen Verfahren kann einfach überprüft werden, ob der ermittelte Füllstandswert dadurch verfälscht wurde, dass eine Person die Messelektroden oder die Abschirmung im Bereich der Messelektroden berührt hat oder den Messelektroden hinreichend nahe gekommen ist, um eine Verfälschung zu verursachen.

Zur genauen Bestimmung des Füllstandes kann vorgesehen sein, dass der Füllstandwert und/oder eine Aussage über die Gültigkeit des Füllstandswertes durch codierte elektromagnetischer Datenübertragung, insbesondere durch Lastmodulation, an ein externes Datenkommunikationsgerät übertragen wird.

Zum selben Zweck kann vorgesehen sein, dass jeweils die Kapazitäten einer Vielzahl von, insbesondere drei, Paaren von Messelektroden, die einander im Außenbereich des Behälters gegenüberliegen, ermittelt werden und der Füllstandswert aufgrund der Kapazitäten ermittelt wird.

Eine besonders genaue Detektion wird ermöglicht, indem
a) für eine Anzahl von Füllständen jeweils Referenzvektoren umfassend die Kapazitäten zwischen den einzelnen Paaren von Messelektroden als Komponenten zur Verfügung gestellt werden, und
b) jedem dieser Vektoren der jeweilige Füllstand zugeordnet wird,
c) ein Vektor umfassend die einzelnen ermittelten Kapazitäten ermittelt wird,
d) eine Anzahl von Referenzvektoren gesucht wird, die vom Vektor den geringsten, insbesondere euklidischen, Abstand aufweisen,
e) eine Interpolationsfunktion gebildet wird, die bei Anwendung auf die in Schritt b) aufgefundenen Referenzvektoren den jeweiligen, diesen Referenzvektoren zugeordneten Füllstand ergibt,
f) die Interpolationsfunktion auf den Vektor angewendet wird und das Ergebnis als Füllstand herangezogen wird.

Mehrere bevorzugte Ausführungsformen der Erfindung werden anhand der folgenden Zeichnungsfiguren näher erläutert.

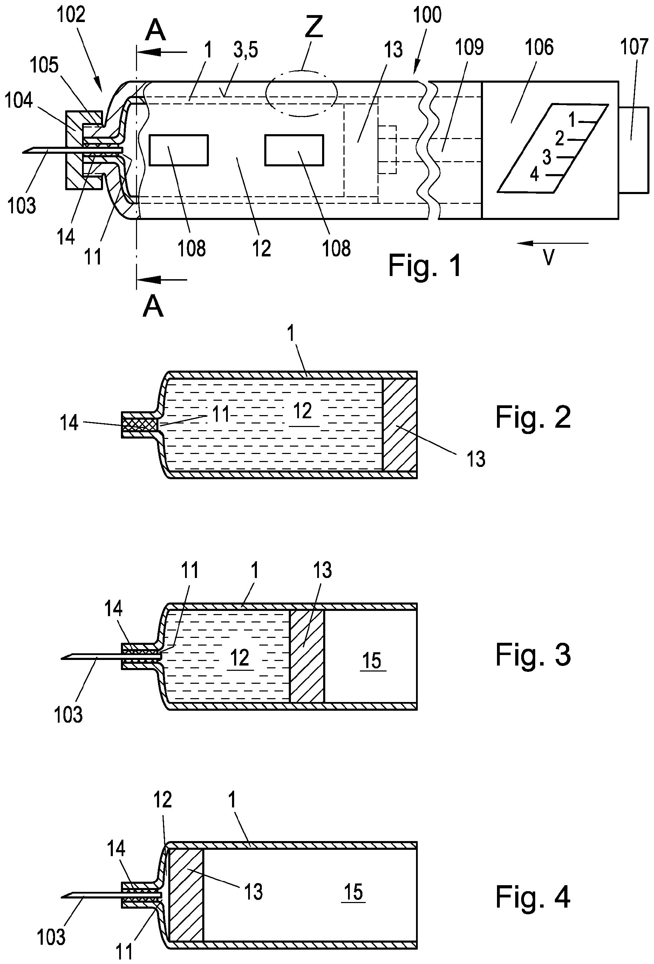

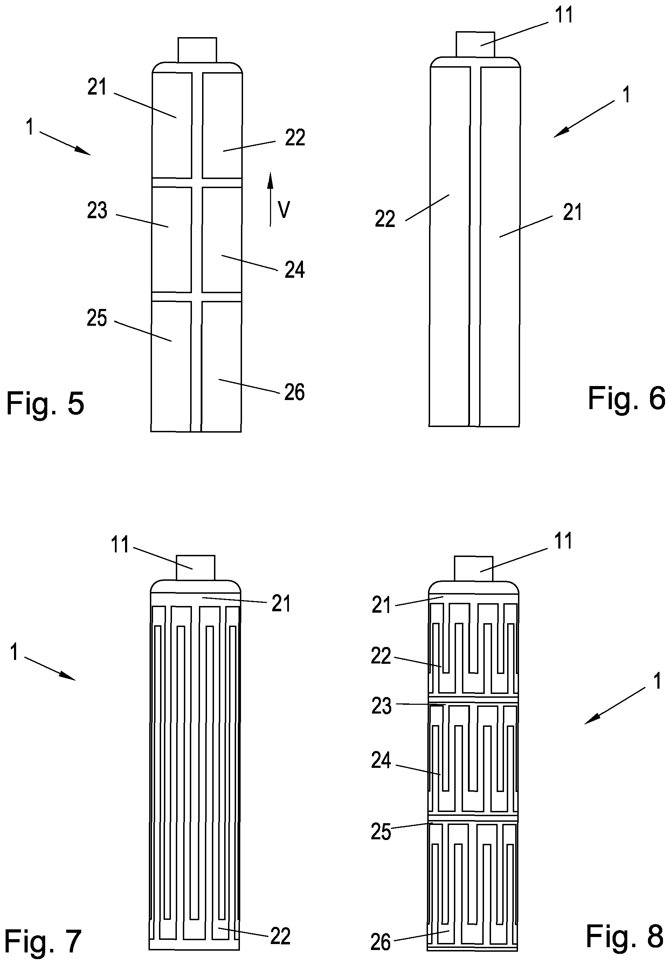

In **Fig. 1** ist eine erste Ausführungsform einer erfindungsgemäßen Abgabevorrichtung in Seitenansicht dargestellt. **Fig. 2** zeigt einen vollständig gefüllten Behälter in Form einer Ampulle in Seitenansicht. **Fig. 3** zeigte einen teilweise entleerten Behälter in Seitenansicht. **Fig. 4** zeigt einen vollständig entleerten Behälter in Seitenansicht. **Fig. 5** zeigt eine alternative Ausführungsform eines Behälters mit drei Paaren von Messelektroden. **Fig. 6** zeigt eine zweite Ausführungsform der Erfindung mit einem einzigen Paar von Messelektroden. **Fig. 7** zeigt eine weitere Ausführungsform der Erfindung mit einem Paar von kammförmig angeordneten Messelektroden. **Fig. 8** zeigt eine weitere Ausführungsform der Erfindung mit drei kammförmig angeordneten Paaren von Messelektroden.

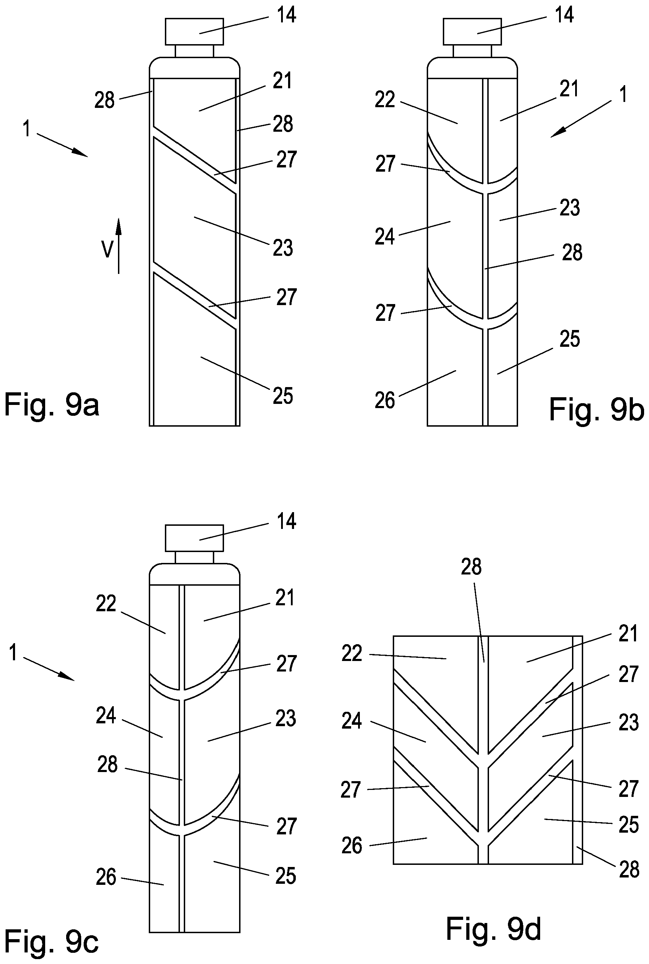

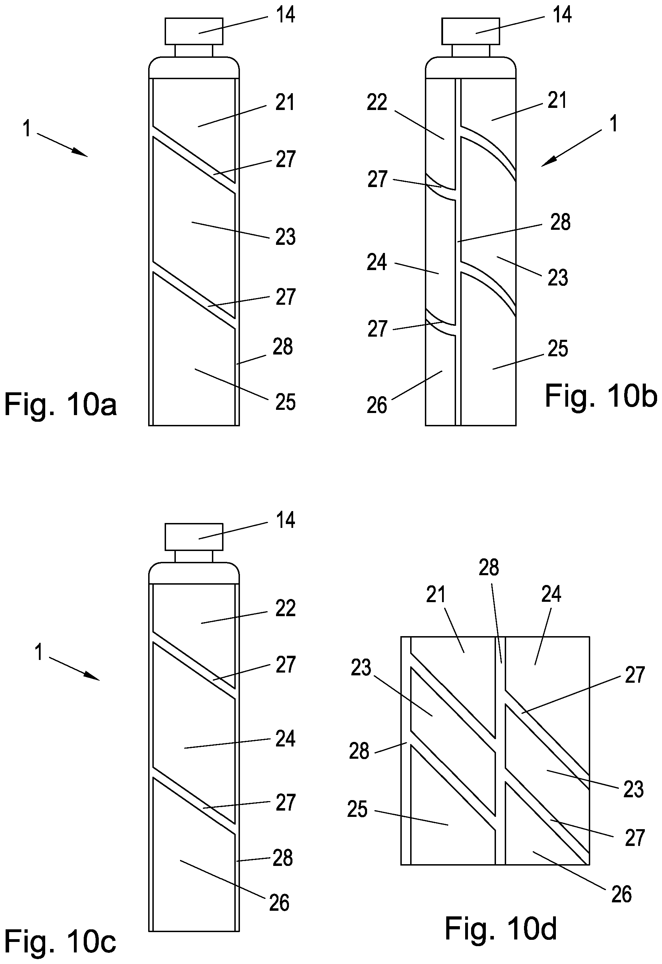

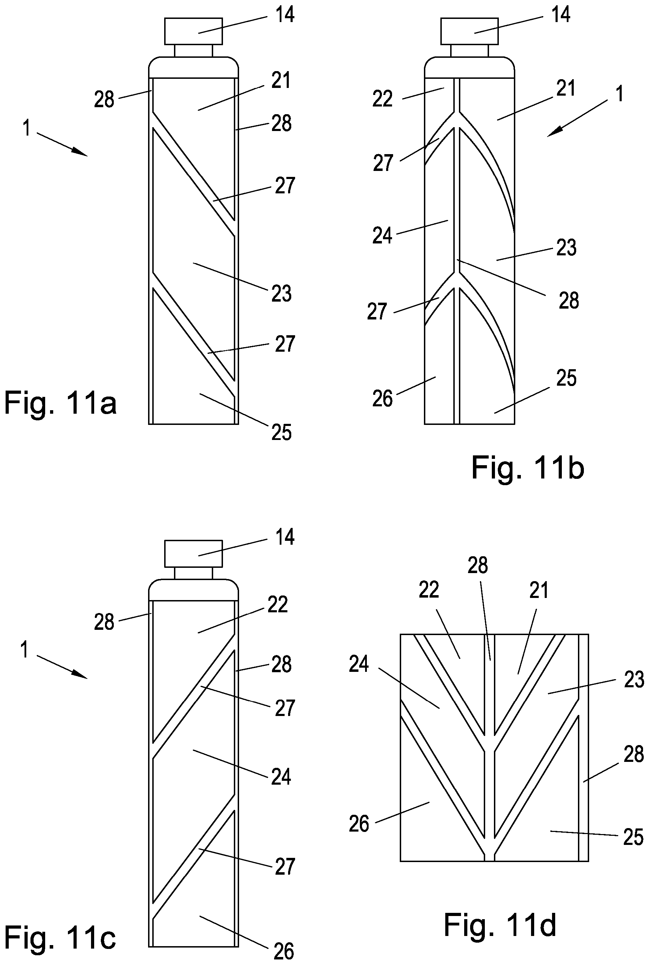

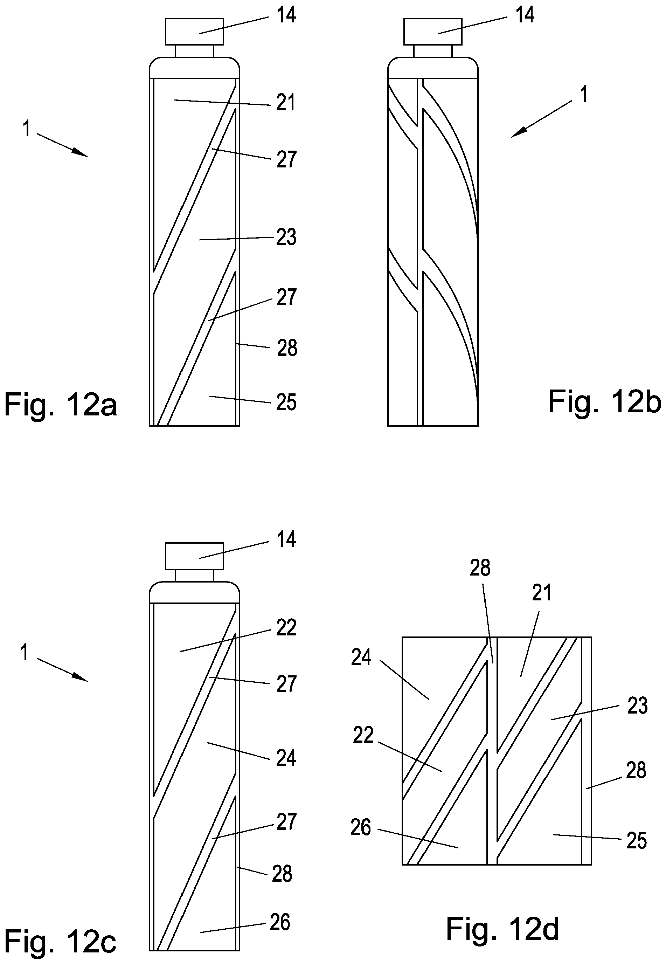

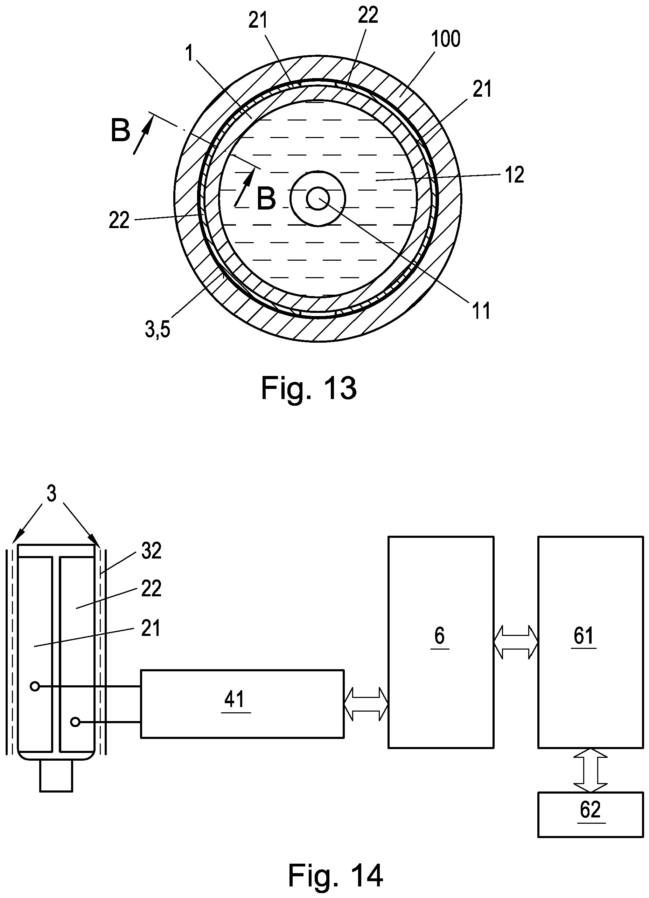

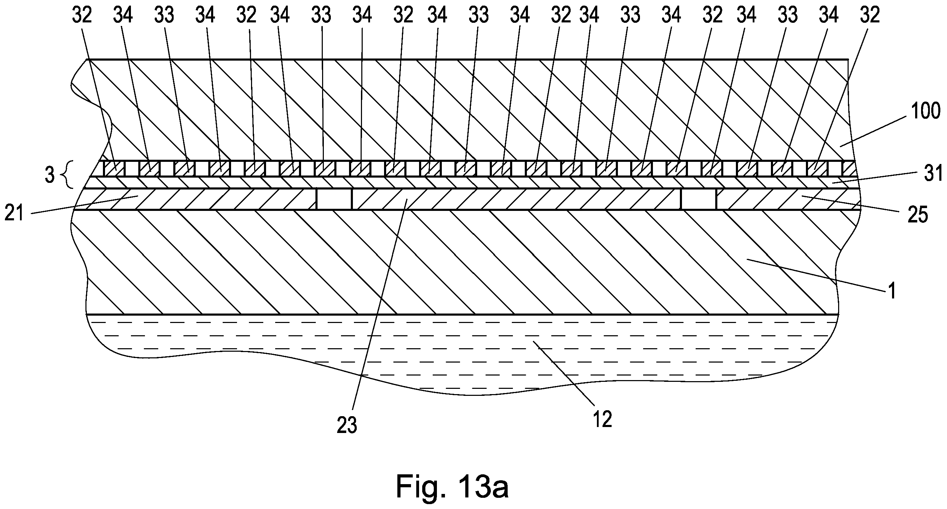

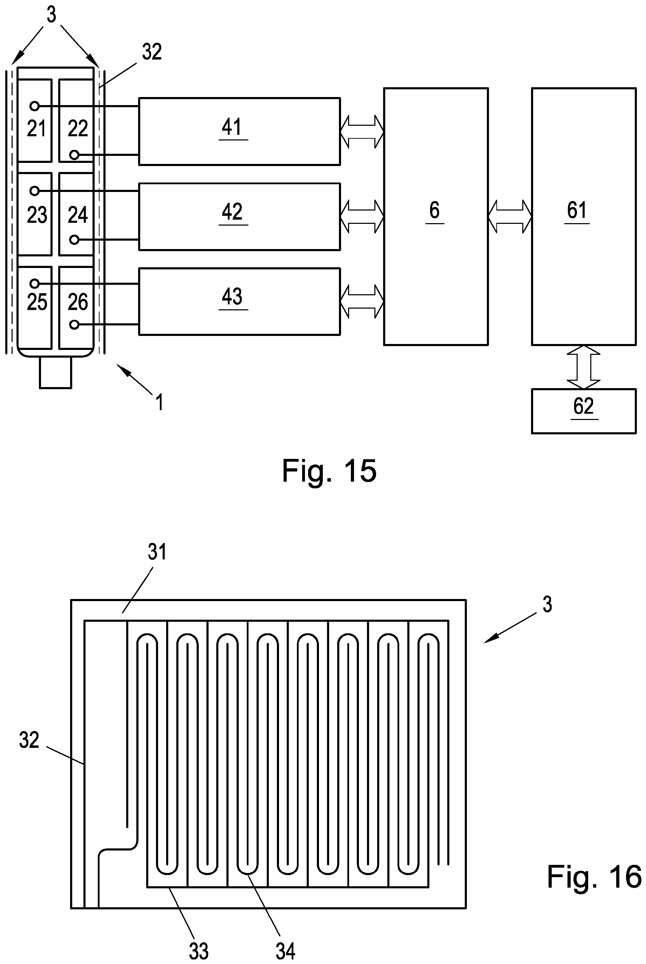

Die **Fig. 9a bis 12d** zeigen weitere Ausführungsformen von Behältern mit schräg verlaufenden Elektroden. **Fig. 13** zeigt ein Ausführungsbeispiel einer erfindungsgemäßen Vorrichtung im Querschnitt. **Fig. 13a** zeigt ein Detail aus **Fig. 1****.** **Fig. 14** **und** **Fig. 15** zeigen zwei Einrichtungen zur Bestimmung des Füllstands innerhalb des Behälters sowie zur Übertragung des ermittelten Füllstands an ein externes Datenkommunikationsgerät.

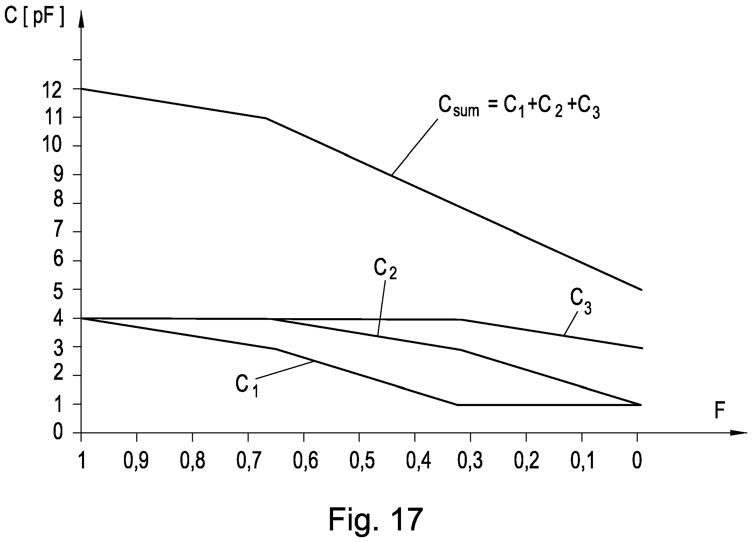

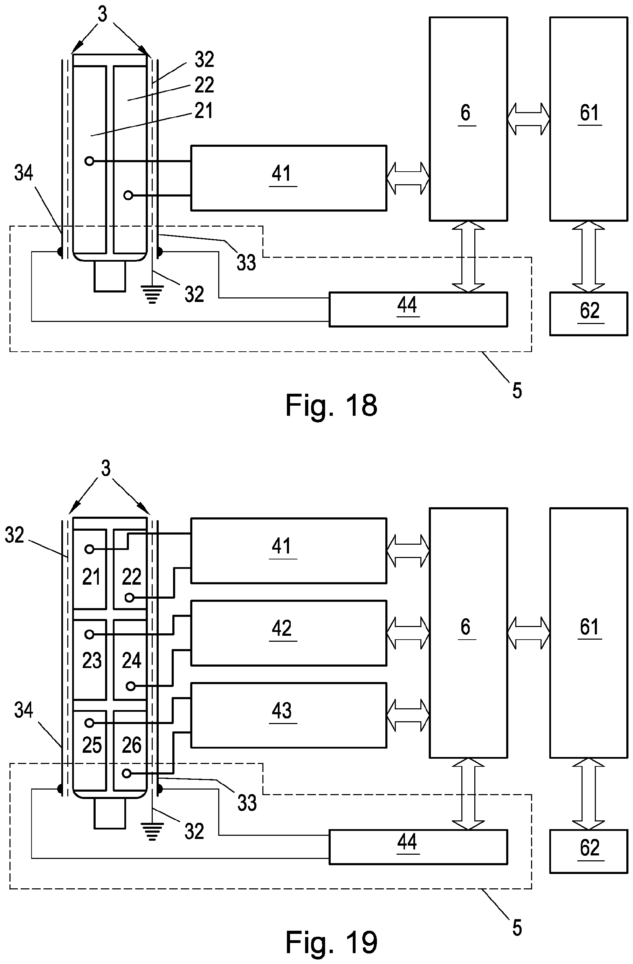

**Fig. 16** zeigt eine Abschirmung in Form einer Folie mit darauf angeordneten Leitern. **Fig. 17** zeigt den theoretischen Verlauf der einzelnen Teilkapazitäten während der Entleerung des Behälters bei der in **Fig. 15** dargestellten Ausführungsform. Die **Fig. 18 und 19** zeigen die in den **Fig. 14** **und** **15** dargestellten Ausführungsformen, wobei zusätzlich eine Berührungsdetektion vorgesehen ist.

In **Fig. 1** ist eine Ausführungsform einer erfindungsgemäßen Abgabevorrichtung 100 in Seitenansicht dargestellt. Die dargestellte Abgabevorrichtung 100 weist einen Behälter 1 auf, der mit einem flüssigen Medikament 12 gefüllt ist. Im vorliegenden Fall wird als flüssiges Medikament 12 Insulin verwendet, es ist jedoch auch möglich, andere flüssige Medikamente 12 wie etwa Hormonpräparate (z.B. Wachstumshormone, ...), Biopharmazeutika oder im Zuge therapeutischer Maßnahmen in der Fortpflanzungsmedizin verwendete Medikamente in den Behälter 1 zu füllen und anschließend auf dieselbe Art zu verabreichen.

Die Abgabevorrichtung 100 weist die Form eines Schreibstifts oder Kugelschreibers auf und kann von einem Patienten bei der Verabreichung der im Behälter 1 befindlichen Flüssigkeit 12 bequem in der Hand gehalten werden. Der Behälter 1 hat die Form einer Patrone oder Ampulle und befindet sich in einem Endbereich 102 der Abgabevorrichtung 100.

Der Behälter 1, der im Detail in **Fig. 2** dargestellt ist, weist an einem Ende, das in diesem Endbereich 102 der Abgabevorrichtung 100 liegt, eine Öffnung 11 zur Abgabe der Flüssigkeit 12 auf. Am gegenüberliegenden Ende weist der Behälter 1 einen Kolben 13 auf, der im Behälter 1 verschiebbar gelagert ist. Hierfür weist der Behälter 1 ein Innenvolumen auf, das abgesehen vom Bereich der Öffnung 11 einen konstanten Querschnitt aufweist. Der Kolben 13 verschließt den Behälter 1 von der der Öffnung 11 gegenüberliegenden Seite her, sodass die im Behälter 1 befindliche Flüssigkeit 12 dicht im Behälter 1 eingeschlossen ist und ausschließlich durch die Öffnung 1 entweichen kann. Im vorliegenden Ausführungsbeispiel weisen der Innenbereich des Behälters 1 sowie der Kolben 13 einen kreisförmigen Querschnitt auf und verfügen über eine im wesentlichen zylindrische Innenwand bzw. Außenwand. Wird der Kolben 13 in den Behälter 1 vorgeschoben, so kann die im Behälter 1 befindliche Flüssigkeit 12 durch die Öffnung 11 aus dem Behälter 1 entweichen. Bei Vorschub des Kolbens 13 in Richtung der Öffnung 11 wird die Flüssigkeit 12 durch die Öffnung 11 aus dem Behälter 1 abgegeben. Die Öffnung 11 des Behälters 1 ist jedoch vor dem Gebrauch, wie in **Fig. 2** dargestellt, durch durch ein Versiegelungselement 14 verschlossen, sodass die Flüssigkeit 12 aus dem Behälter 1 nicht entweichen kann.

**Fig. 3** zeigt den in **Fig. 2** dargestellten Behälter 1, nachdem ein Teil der Flüssigkeit 12 durch die Öffnung 11 über eine Injektionsnadel 103 appliziert wurde.
**Fig. 4** zeigt den in **Fig. 2** dargestellten Behälter 1, nachdem die Flüssigkeit 12 aus dem Behälter 1 durch die Öffnung 11 über eine Injektionsnadel 103 vollständig entleert wurde.
In den Darstellungen der **Fig. 3** und **Fig. 4** befindet sich der Kolben 13 in einer mittleren Position bzw. in Endstellung, d.h. der Behälter 1 ist teilweise (**Fig. 3**) bzw. vollständig (**Fig. 4**) entleert. Im Bereich 15 hinter dem Kolben 13 befindet sich Luft. Die Abgabevorrichtung 100 weist weiters im Bereich der Öffnung 11 des Behälters 1 eine Injektionsnadel 103 auf, die einerseits das Versiegelungselement 14 durchdringt und in das Innere des Behälters 1 ragt und andererseits von der Abgabevorrichtung 1 absteht.

Wie in **Fig. 1** dargestellt, ist die Injektionsnadel 103 in diesem Ausführungsbeispiel mit einem Gehäuse 104 verbunden, das auf die Abgabevorrichtung 100 aufgeschraubt ist. Die Abgabevorrichtung 100 weist ein Außengewinde 105 auf, das an ein gegengleich geformtes passendes Innengewinde des Gehäuses 104 angepasst ist. Wird der Kolben 13, wie in **Fig. 3** dargestellt, in Richtung der Öffnung 11 verschoben, so kann die im Inneren des Behälters 1 befindliche Flüssigkeit durch die Öffnung 11 und die Injektionsnadel 103 dem jeweiligen Patienten verabreicht werden. Das Gehäuse der Abgabevorrichtung 100 weist zwei Sichtöffnungen 108 auf, um den Füllstand F der im Behälter 1 befindlichen restliche Flüssigkeit 12 visuell bestimmen zu können.

Darüber hinaus weist die Abgabevorrichtung 100 (**Fig. 1**) eine Einstelleinheit 106 auf, mit der eine bestimmter Vorschub des Kolbens 13 und - damit korrespondierend - eine bestimmte Menge der abzugebenden Flüssigkeit 12 voreingestellt werden kann. Nach der Einstellung der Menge der abzugebenden Flüssigkeit 12 wird mittels Druckbetätigung durch den Patienten auf eine Betätigungseinheit 107 ein Vorschubelement 109 gegen den Kolben 13 des Behälters 1 gedrückt. Der Kolben 13 wird in den Behälter 1 geschoben und die im Behälter 1 befindliche Flüssigkeit 12 wird über die Injektionsnadel 103 an den Patienten verabreicht. Das Vorschubelement 109 ist gegen ein Zurücksetzen entgegen der Vorschubrichtung V des Kolbens 13, d.h. von der Öffnung 11 weg, gesichert, sodass der Kolben 13 nur noch weiter in Richtung der Öffnung 11 bewegt werden kann.

Besonders vorteilhaft ist die Verwendung eines Behälters 1 mit drei Paaren von Messelektroden, wie in **Fig. 5** dargestellt. Der Behälter 1 weist, wie in **Fig. 5** dargestellt, drei Paare von Messelektroden 21-26 auf. Sämtliche Messelektroden 21-26 sind im Außenbereich des Behälters 1, im vorliegenden Fall auf der Außenwand des Behälters 1 angeordnet. In diesem bevorzugten Ausführungsbeispiel der Erfindung liegen je zwei einander zugeordnete Messelektroden 21-26 einander in Umfangsrichtung beabstandet an der Außenwand des Behälters 1 gegenüber. Die einzelnen Paare einander zugeordneter Messelektroden 21-26 sind zueinander in Vorschubrichtung V des Kolbens 13 beabstandet. Die Messelektroden 25, 26 des dritten Paars liegen von der Öffnung 11 des Behälters 1 am weitesten entfernt. Die Messelektroden 21, 22 des ersten Paars liegen der Öffnung 11 am nächsten. Die Messelektroden 23, 24 des zweiten Elektrodenpaars liegen - in Vorschubrichtung V des Kolbens 13 gesehen - zwischen den Messelektroden 21, 22; 25, 26 des ersten und des dritten Paars. Die Messelektroden 21-26 liegen in einem Bereich der Außenwand des Behälters 1 flächig an dieser an. In dem in **Fig. 5** dargestellten Ausführungsbeispiel weisen die Messelektroden 21-26 eine rechteckige Form auf. Die Messelektroden 21-26 erstrecken sich über den gesamten Vorschubbereich des Kolbens 13. Werden mehrere Elektrodenpaare verwendet, kann es vorteilhaft sein, wenn die Ausdehnung eines Elektrodenpaars in Vorschubrichtung V des Kolbens 13 der Ausdehnung des Kolbens 13 in seiner Vorschubrichtung V entspricht.

Alternativ können jedoch auch andere Elektrodenformen, etwa kreisförmige oder kammartige Elektrodenformen für die Messelektroden 21-26 verwendet werden. Die Verwendung mehrerer Paare von Messelektroden 21-26 ist im Sinne einer genauen Messung des Flüssigkeitsinhalts oder Flüssigkeitsstands in länglichen Behältern 1 zwar grundsätzlich vorteilhaft, aber gerade bei kurzen oder kompakten Behältern 1 nicht erforderlich. In einem in **Fig. 6** dargestellten alternativen Ausführungsbeispiel eines Behälters 1 ist lediglich ein einziges Paar von Messelektroden 21, 22 vorgesehen, die länglich und sich über den gesamten Vorschubbereich erstreckend ausgebildet sind. Die beiden Messelektroden 21, 22 liegen einander umfangsseitig auf derselben Höhe hinsichtlich der Vorschubrichtung V des Kolbens 13 gegenüber.

Darüber hinaus ist es auch möglich, unterschiedliche Formen von Messelektroden 21-16 zu verwenden. Eine vorteilhafte Ausführungsform sieht vor, dass die Messelektroden 21-26 als Kammelektroden oder interdigitale Elektroden ausgebildet sind. Die Messelektroden 21-26 sind einander paarweise zugeordnet und weisen eine Kammstruktur auf, wobei die Zähne einander zugeordneter Messelektroden 21, 22; 23, 24; 25, 26 ineinander greifen. Wie in **Fig. 7** und **Fig. 8** dargestellt, können Kammelektroden sowohl für eine Anordnung mit einem (**Fig. 7**) als auch mit mehreren Paaren von Messelektroden 21, 22; 23, 24; 25, 26 verwendet werden.

Je nach Anwendungsfall ist es auch möglich, unterschiedlich große Messelektroden 21-26 vorzusehen, um eine besonders vorteilhafte Bestimmung des Füllstandes F im Behälter 1 zu ermöglichen. Besonders vorteilhaft ist die Verwendung von parallelogrammförmigen bzw. dreieckigen Messelektroden 21-26, bei denen die Elektroden durch Trennbereiche 27 voneinander getrennt sind, die zur Vorschubrichtung V des Kolbens bzw. der Längsachse des Behälters 1 im Winkel verlaufen, beispielsweise in einem Winkel von 45°. Bei einer solchen Anordnung ergibt sich ein fließender Übergang, sodass eine besonders genaue Bestimmung des Füllstandes F möglich wird. Die **Fig. 9a bis 12d** zeigen vier unterschiedliche Ausführungsformen mit Trennbereichen 27 zwischen den Messelektroden 21-26, die im Winkel zur Vorschubrichtung V stehen. Weiters ist bei diesen Ausführungsformen ein achsparalleler Trennbereich 28 vorgesehen, der jeweils einander zugeordnete Paare von Messelektroden 21, 22; 23, 24; 25, 26 voneinander trennt.

Mit allen solchen Elektrodenanordnungen ist es möglich, aufgrund der Kapazität zwischen den Messelektroden 21-26 auf den Füllstand F des Behälters 1 zu schließen. Um eine möglichst präzise Messung der einzelnen Kapazitäten C₁, C₂, C₃ zu ermöglichen und damit einen Rückschluss auf den Füllstand F des Behälters 1 ziehen zu können, sieht die Erfindung bei einer Abgabevorrichtung vor, eine elektrische Abschirmung 3 für elektrische Felder außerhalb der Messelektroden 21-26 mantelförmig um den Behälter 1 anzuordnen. In **Fig. 13** ist ein Schnitt durch den Behälter 1 dargestellt, der die Abschirmung 3, die Messelektroden 21, 22, die Wand des Behälters und die Flüssigkeit 12 im Inneren des Behälters 1 darstellt. Die Abschirmung 3 bewirkt, dass die zwischen den Elektroden 21, 22 gemessene Kapazität nicht oder nur in vernachlässigbarem Ausmaß verfälscht wird, wenn eine Person die Abgabevorrichtung 100 berührt bzw. ihr nahekommt und dadurch die an den Messelektroden 21, 22 herrschenden elektrischen Feldbedingungen verändert. In einem ersten Ausführungsbeispiel der Erfindung ist die Abschirmung 3 als Folie aus elektrisch leitfähigem Material, beispielsweise als Kupferfolie mit einer Dicke von 50 µm ausgebildet, die mantelförmig um den Behälter 1 und die an diesem anliegenden Messelektroden 21, 22 gewickelt ist. Die Messelektroden 21, 22 und die Abschirmung 3 sind voneinander getrennt und sind nicht leitend miteinander verbunden. Die Abschirmung 3 dient zur Unterdrückung des Einflusses von äußeren Beeinflussungen, z.B. Änderungen der Permittivität sowie elektrischen Feldern im unmittelbaren Außenbereich der Messelektroden 21, 22. Die Abschirmung 3 umgibt sowohl die Messelektroden 21, 22 als auch den Behälter 1 und befindet sich vorteilhafterweise nicht zwischen den Messelektroden und dem Behälter 1.

**Fig. 13a** zeigt das Detail Z aus **Fig. 1** im Schnitt B-B von **Fig. 13****.** Deutlich zu erkennen ist - wenn auch maßstäblich nicht korrekt - die Anordnung der Wand des Behälters 1 gegenüber den Elektroden 21, 23, 25 sowie der Abschirmung 3. Die einzelnen Leiter 32-34 auf der Folie 3 sind im Schnitt dargestellt. Außerhalb der Abschirmung 3 befindet sich das Gehäuse der Abgabevorrichtung 100.
Alternativ ist es auch möglich, die Abschirmung 3 unmittelbar außerhalb der Außenwand der Abgabevorrichtung 100 und/oder außerhalb eines den Behälter 1 zumindest teilweise umschließenden Trägers anzuordnen.

Zur Bestimmung des momentanen Füllstandes F der Flüssigkeit 12 in Behälter 1 wird zunächst die vorhandene Kapazität zwischen den Messelektroden 21, 22 bestimmt. In **Fig. 14** ist eine Messanordnung zur Bestimmung der Kapazität eines einzigen Paars von Messelektroden 21, 22 dargestellt. **Fig. 15** zeigt eine Messanordnung bei der Verwendung mehrerer Paare von Messelektroden 21-26. In **Fig. 14** **und** **15** ist jeweils eine Recheneinheit 6 in Form eines Mikrocontrollers vorgesehen, dem eine bzw. drei Kapazitätsmesseinrichtungen 41, 42, 43 vorgeschaltet sind. Jedem Paar von Messelektroden 21-26 ist jeweils eine der in **Fig. 15** dargestellten Kapazitätsmesseinrichtungen 41, 42, 43 zugeordnet. Die Messelektroden 21-26 sind jeweils mit den Anschlüssen der Kapazitätsmesseinrichtungen 41, 42, 43 verbunden. Am Ausgang der Kapazitätsmesseinrichtungen 41, 42, 43 liegt jeweils ein der jeweiligen Kapazität des jeweiligen Elektrodenpaars entsprechender, diese repräsentierender bzw. zu dieser proportionaler Kapazitätsmesswert C₁, C₂, C₃ an, der an die Recheneinheit 6 übertragen wird. Aufgrund der einzelnen übertragenen Kapazitätsmesswerte C₁, C₂, C₃ ermittelt die Recheneinheit 6 aufgrund eines später beschriebenen Kalibriervorgangs einen Wert für den Füllstands F. Die Recheneinheit 6 hält diesen Wert an ihrem Ausgang zur Verfügung. Dieser Wert kann insbesondere auf Anfrage über eine der Recheneinheit 6 nachgeschaltete Antenne 62 an ein (nicht dargestelltes) externes Datenkommunikationsgerät übertragen werden.

Selbstverständlich kann die Zahl der verwendeten Paare von Messelektroden 21-26 an die Anforderungen an die Genauigkeit der Messung angepasst werden. Insbesondere ist es auch möglich, ein einziges Paar von Messelektroden 21, 22 zu verwenden und nur den zwischen diesen Messelektroden 21, 22 ermittelten Kapazitätsmesswert C₁ zur Bestimmung des Füllstands F heranzuziehen. (**Fig. 15**)

Der Recheneinheit 6 ist ein Kommunikationscontroller 61 nachgeschaltet, der an eine Antenne 62, im vorliegenden Fall eine Spulenantenne, angeschlossen ist. Der Kommunikationscontroller 61 ermöglicht die Übertragung des ermittelten Füllstandes F an ein externes Datenkommunikationsgerät. Darüber hinaus kann auch noch vorgesehen sein, dass das externe Datenkommunikationsgerät über die Antenne 62 elektrische Energie an den Kommunikationscontroller 61, die Recheneinheit 6, sowie an die Kapazitätsmesseinrichtungen 41-43 überträgt, sodass die gesamte in **Fig. 14** bzw. **Fig. 15** dargestellte Schaltung ohne separate Energieversorgung auskommt.

Im Folgenden wird die konkrete Ermittlung des Füllstandes F der Flüssigkeit 12 im Behälter 1 anhand der ermittelten Kapazitätsmesswerte C₁, C₂, C₃ näher dargestellt. In **Fig. 17** ist jeweils die Abhängigkeit der einzelnen Kapazitätsmesswerte C₁, C₂, C₃ vom Füllstand F bei der in **Fig. 5** dargestellten Ausführungsform eines erfindungsgemäßen Behälters 1 schematisch dargestellt. Am Beginn des Entleervorgangs des Behälters 1 befindet sich zunächst ausschließlich die Flüssigkeit 12 zwischen den Messelektroden 21-26. Im Zuge der Entleerung gelangt der Kolben 13 zunächst in den Zwischenbereich zwischen die Messelektroden 21, 22 des ersten Messelektrodenpaars, sodass sich aufgrund der geringeren Permittivität des Kolbens 13 gegenüber der Flüssigkeit 12 ein kontinuierliches Absinken des Kapazitätsmesswertes C₁ des ersten Messelektrodenpaars zu beobachten ist. Nachdem der Kolben 13 durch den Zwischenbereich zwischen den Messelektroden 21, 22 des ersten Messelektrodenpaars geschoben wurde, befindet sich zwischen den beiden Messelektroden 21, 22 des ersten Messelektrodenpaars Luft 15. Aufgrund der noch geringeren Permittivität der Luft zwischen den beiden Messelektroden 21, 22 des ersten Messelektrodenpaars sinkt der zwischen diesen Messelektroden 21, 22 gemessene Kapazitätsmesswert C₁ noch weiter ab. Ein ähnliches Verhalten ist auch für die Kapazitätsmesswerte C₂, C₃ zwischen den Messelektroden 23-26 des zweiten und dritten Messelektrodenpaars bei Entleerung des Behälters 1 zu bemerken.

In einer besonderen Ausführungsform der Erfindung kann die Summe Cₛᵤₘ der einzelnen Kapazitätsmesswerte C₁, C₂, C₃ zur Bestimmung des Füllstands F herangezogen werden. Durch Ermittlung einer Kalibrierkurve kann für eine Anzahl von verschiedenen Füllständen jeweils die zugehörige Summe Cₛᵤₘ der einzelnen Kapazitätsmesswerte C₁, C₂, C₃ ermittelt werden, wobei jedem Füllstand F jeweils eine Summe Cₛᵤₘ zugeordnet wird. Die so erstellten einzelnen Datensätze umfassend jeweils einen Kapazitätsmesswert Cₛᵤₘ und einen Füllstand F werden in einem Kalibrierspeicher in der Recheneinheit 6 abgelegt.

Nach der Messung und Bestimmung der einzelnen Kapazitätsmesswerte C₁, C₂, C₃ wird deren Summe Cₛᵤₘ ermittelt und mit den einzelnen im Kalibrierspeicher abgespeicherten Summen Cₛᵤₘ verglichen. Es wird dasjenige Paar ausgewählt, dessen zugehörige Summe Cₛᵤₘ mit der Summe der ermittelten Kapazitätsmesswerte C₁, C₂, C₃ am besten übereinstimmt. Der der am besten übereinstimmenden Summe Cₛᵤₘ jeweils zugeordnete Füllstand wird als Füllstand F des Behälters 1 angesehen, die Recheneinheit 6 hält diesen Füllstand F an ihrem Ausgang zur Verfügung und gibt den Füllstand F, wie vorstehend beschrieben, auf Anfrage über eine Antenne 62 an ein externes Datenkommunikationsgerät ab.

Die Praxis zeigt, offenbar aufgrund komplexer kapazitiver Verkopplungsphänomene der Messelektroden 21-26 untereinander auch stark abweichende Verläufe der gemessenen Kapazitäten C₁, C₂, C₃ in Abhängigkeit vom Füllstand F, die deutlich von den in **Fig. 17** dargestellten, theoretisch zu erwartenden Verläufen abweichen. Die messbaren Kurvenverläufe sind jedoch sehr gut reproduzierbar und zeigen für jede Kapazität C₁, C₂, C₃ unterschiedliche Steilheiten in unterschiedlichen Kurvenabschnitten bzw. Füllstandsbereichen, wobei entgegen den theoretischen Erwartungen die größte Steilheit des Kurvenverlaufs bzw. die größte Kapazitätsveränderung nicht zwangsläufig zwischen denjenigen Messelektroden 21-26 auftritt zwischen denen sich gerade der Flüssigkeitsspiegel befindet. Da größere Kurvensteilheit aber bessere Messauflösung/Messgenauigkeit bedeutet, kann für die Berechnung des Füllstands alternativ zur Bildung einer einfachen Summe der drei Einzel-Kapazitätsmesswerte eine gewichtete Summe verwendet werden, wobei für jeden der drei Summanden in jedem Kurvenabschnitt separat ein eigenes Gewicht im Zuge der Kalibrierung ermittelt wird.

Um eine Umrechnung zwischen einzelnen Kapazitäten C₁, C₂, C₃ und einem Füllstand F zu erreichen, wird eine Kalibrierung vorgenommen, bei der der mit dem Medikament gefüllte Behälter 1 oder ein baugleicher Referenzbehälter entleert wird. Während der Entleerung werden jeweils der Füllstand F sowie die einzelnen Kapazitäten C₁, C₂, C₃ ermittelt. Für jeden der bei der Entleerung eingenommenen Füllstände F stehen somit jeweils einzelne Kapazitätswerte C₁, C₂, C₃ zur Verfügung. Im vorliegenden Ausführungsbeispiel werden 30 äquidistande Füllstände F bei der Entleerung eingenommen, wobei der Ausgangszustand mit 1 und der vollständig entleerte Zustand mit 0 bezeichnet wird. Die Kapazitätswerte C₁, C₂, C₃ werden jeweils in einem Referenzvektor V_{ref} abgespeichert, der dem jeweiligen Füllstand F sowie den jeweiligen Gewichten a, b, c zugeordnet wird. Für jeden Füllstand F steht somit ein Referenzvektor V_{ref} zur Verfügung. Die Gewichte werden durch Optimierung derart festgelegt, dass die gewichtete Summe a . C₁ + b . C₂ + c . C₃ eine lineare Annäherung für den Füllstand F darstellt.

Will man nun den tatsächlichen Füllstand F anhand von durch Messung ermittelten Kapazitätswerten C₁, C₂, C₃ bestimmen, so kann dies aufgrund der während der Kalibrierung ermittelten Gewichte vorgenommen werden, wobei für jede Messung jeweils so viele Gewichte zur Verfügung stehen, wie Kapazitätswerte C₁, C₂, C₃ ermittelt wurden. Zunächst wird aufgrund der ermittelten bzw. gemessenen Kapazitätswerte C₁, C₂, C₃ ein Vektor Vₘₑₛₛ [C₁, C₂, C₃] erstellt, der die Kapazitätswerte C₁, C₂, C₃ als Komponenten aufweist. Anschließend wird der Vektor Vₘₑₛₛ mit den ermittelten Referenzvektoren V_{ref} verglichen und derjenige Referenzvektor gesucht, der zum Vektor Vₘₑₛₛ den geringsten Abstand aufweist. Im vorliegenden Ausführungsbeispiel wird als Abstandsmaß der Euklidische Abstand verwendet. Anschließend werden diejenigen Referenzvektoren V_{ref} ermittelt, der den jeweils nächstkleinsten Abstand zum Vektor Vₘₑₛₛ aufweisen. Es wird eine interpolierende Funktion, beispielsweise eine lineare Interpolationsfunktion bestimmt, die bei Anwendung auf die durch Kalibrierung ermittelten Referenzvektoren V_{ref} den jeweils diesen zugeordneten Füllstand F zurückliefert. Die Kapazitätswerte C₁, C₂, C₃ werden in die Interpolationsfunktion eingesetzt und man erhält einen gemittelten Füllstandswert.

Die Antenne 62 kann aus Platzgründen vorteilhafterweise außen auf der Abschirmung 3 angeordnet sein. Um eine vorteilhafte Kombination zu gewährleisten, weist die Abschirmung 3 eine Folie 31 aus einem elektrisch und magnetisch nicht leitfähigen Material, wie z.B. Kunststoff auf. Auf der Folie 31, dargestellt in **Fig. 16****,** sind Leiter 32-34 in Form von Leiterbahnen aufgebracht. Wenn die Leiter 32-34 auf der Folie 31 derart ausgebildet sind, dass keine großflächig geschlossenen Leiterschleifen existieren, in denen sich Wirbelströme ausbilden können, werden die vom externen Datenkommunikationsgerät abgegebenen elektromagnetischen Wellen von der Abschirmung 3 nicht maßgeblich beeinflusst und können von der Antenne 62 empfangen werden. Weiters ist es dadurch auch möglich, Energie in Form elektromagnetischer Wellen an die Antenne 62 zu übertragen, die ausreicht, um an die Antenne angeschlossene elektrische Bauteile ausreichend mit Energie zu versorgen.

Sofern eine zusätzliche Genauigkeit bei der Bestimmung des Füllstandes F im Inneren des Behälters 1 erforderlich ist, kann vorgesehen sein, dass ein Messwert für den Füllstand F dann invalidiert bzw. für ungültig erklärt wird, wenn das elektrische Feld im Außenbereich des Behälters 1, z.B. durch Berührungen bzw. das Nahekommen elektrisch leitfähiger Körper oder Körper mit hoher dielektrischer Permittivität verfälscht wurde.

Die Abschirmung 3 weist eine elektrisch und magnetisch nicht leitfähige Folie 31 auf, auf der eine Vielzahl von Leitern 32, 33, 34 durch Beschichtung ausgebildet sind. Die Folie 31 besteht im vorliegenden Ausführungsbeispiel aus biegsamem Kunststoff. Die Leiterbahnen weisen eine Schichtdicke von ca. 50 µm und eine Breite von etwa 1000 µm auf. Vorteilhaft sind Breiten der Leiter 32-34 zwischen 100 µm und 3000 µm.

Um die Ausbildung von Wirbelströmen und dadurch eine Beeinträchtigungen einer NFC-Kommunikation zu vermeiden, kann die Breite der Leiter 32-34 auf weniger als 3 mm beschränkt werden. Darüber hinaus können die Leiter 32-34, wie in **Fig. 16** dargestellt, schleifenfrei, d.h. frei von geschlossenen Leiterschleifen, ausgebildet sein, d.h. keine geschlossenen Schleifen umfassen, um die Ausbildung von Wirbelströmen in hinreichendem Maße zu verhindern und eine Beeinträchtigung einer NFC-Kommunikation zu vermeiden, gleichzeitig jedoch auch kapazitive Beeinflussungen der innerhalb der Abschirmung 3 befindlichen Messelektroden 21-26 zu vermeiden.

In diesem besonderen Ausführungsbeispiel der Erfindung sind deshalb zwei der drei Leiter 32, 33 als ineinandergreifende Kammleiter 32, 33 ausgebildet, der dritte Leiter 34 verläuft mäanderförmig zwischen den beiden Kammleitern 32, 33. Neben diesem Ausführungsbeispiel gibt es selbstverständlich auch noch eine Vielzahl weiterer Ausführungsbeispiele der schleifenfreien Anordnung mehrerer miteinander nicht elektrisch verbundenen Leiterbahnen bzw. Elektroden auf der Oberfläche einer Folie 31 oder im Inneren bzw. zwischen einzelnen Lagen einer mehrlagig aufgebauten Folie. Auch können sowohl Vorderseite als auch Rückseite der Folie 31 mit Leitern 32-34 bedruckt sein. Alternativ können auch mehrere mäanderförmige Leiter 34 nebeneinander zwischen den Kammleitern 32, 33 angeordnet werden oder mehrere Leiter 34 spiralenförmig auf der Folie 31 angeordnet sein.

Zwei Leiterbahnen, nämlich einer der beiden Kammleiter 32 sowie der mäanderförmige Leiter 34, werden als Berührungssensor 5 verwendet. Der zweite Kammleiter 33 wird auf ein vorgegebenes Massepotential gelegt und dient als elektrische Abschirmung. Berührt eine Person die Abschirmung 3 oder kommt die Person der Abschirmung 3 nahe, so verändert sich aufgrund der Änderung der Permittivität der Umgebung die Kapazität zwischen den Leitern 32, 34 des Berührungssensors 5. Die Veränderung dieser Kapazität zwischen den Leitern 32, 34 kann mittels einer weiteren Kapazitätsmesseinrichtung 44 bestimmt werden, die Leiter 32, 34 der Abschirmung 3 bzw. des Berührungssensors 5 sind an die Messanschlüsse der weiteren Kapazitätsmesseinrichtung 44 angeschlossen. Diese weitere Kapazitätsmesseinrichtung 44 bestimmt einen weiteren Kapazitätswert C' und leitet diesen, wie in **Fig. 18** bzw. **Fig. 19** dargestellt, an die Recheneinheit 6 weiter. Übersteigt die Veränderung des ermittelten weiteren Kapazitätsmesswertes C' einen vorgegebebenen Schwellenwert T, so wird angenommen, dass der aufgrund der Kapazitätsmesswerte C₁, C₂, C₃ ermittelte Füllstand F aufgrund der Berührung fehlerhaft ist. Der ermittelte Füllstand F wird invalidiert.

Im vorliegenden besonderen Ausführungsbeispiel der Erfindung wird eine Abschirmung 3 verwendet, die zugleich als Berührungsdetektion 5 fungiert und aus dem Kammleiter 32 und dem mäanderförmig verlaufenden Leiter 34 besteht. Aus physikalischer bzw. funktioneller Sicht sind elektrische Schirmung 3 und Berührungsdetektion 5 jedoch zwei völlig getrennte und unterschiedliche Einheiten, die sich durch die in **Fig. 16** dargestellte konkrete Anordnung besonders vorteilhaft, nämlich in einer Ebene druckbar, realisieren lässt. Diese funktionelle Trennung von elektrischer Schirmung 3 und Berührungsdetektion 5 ist selbstverständlich ohne weiteres möglich. Nur zur einfacheren Darstellung wurden die in einer Ebene der Folie 31 liegenden Leiter 32, 33, 34 der Schirmung 3 bzw. der Berührungsdetektion 5 in den **Fig. 14****,** **15****,** **18, 19** nebeneinander dargestellt.

Eine alternative Ausführungsform der Erfindung ermöglicht das Austauschen des Behälters 1 aus der Abgabevorrichtung 100. Außerhalb des Behälters 1 zwischen dem Behälter 1 und der Abschirmung 3 ist ein nicht dargestellter Träger angeordnet. Auf diesem befinden sich Messelektroden 21-26. Der Träger liegt am Behälter 1 an und ist vorteilhafterweise durch einen Teil des Gehäuses der Abgabevorrichtung 100 ausgebildet. Die Messelektroden 21-26 sind auf der am Behälter 1 anliegenden Wand des Trägers angeordnet. Das Gehäuse der Abgabevorrichtung 100 lässt sich öffnen und der Behälter 1 kann aus dem Gehäuse der Abgabevorrichtung 100 entfernt werden. Der Träger bildet einen Teil der Abgabevorrichtung 100.

Vorteilhafterweise können der Kommunikaitonscontroller 61, die Recheneinheit 6, die Kapazitätsmesseinrichtungen 41-44 sowie die Antenne 62 auf der Folie 31 angeordnet sein.

## Patentansprüche

1. Abgabevorrichtung zur Abgabe von Flüssigkeiten (12), insbesondere von flüssigen Medikamenten an Personen, umfassend
- einen mit der Flüssigkeit (12) gefüllten Behälter (1), der an einem Ende eine Öffnung (11) zur Abgabe der Flüssigkeit (12) aufweist,
- zumindest eine Paar von im Außenbereich, insbesondere an der Wand, des Behälters (1) einander gegenüberliegend angeordneten kapazitiven Messelektroden (21, 22) zur Bestimmung der Permittivität des jeweiligen Mediums im Zwischenbereich zwischen den Messelektroden, und
- eine die Messelektroden (21, 22) mantelförmig umgebende und um den Behälter (1) angeordnete Abschirmung (3)
wobei die Abschirmung (3) als mit Leitern (32, 33, 34) in Form von Leiterbahnen beschichtete Folie (31) ausgebildet ist
**dadurch gekennzeichnet,**
**dass** auf der Folie (31) drei separate Leiter (32, 33, 34) ausgebildet sind, wobei der erste und der zweite Leiter (32, 33) als ineinandergreifende Kammleiter ausgebildet sind und der dritte Leiter (34) mäanderförmig ausgebildet zwischen den beiden Kammleitern (32, 33) liegt.

2. Abgabevorrichtung nach Anspruch 1, **dadurch gekennzeichnet,**
**dass** der Bereich zwischen der Flüssigkeit (12) und den Messelektroden (21, 22) frei von der Abschirmung (3) ist, und/oder
**dass** die Abschirmung (3) von des Messelektroden (21, 22) beabstandet ist, und/oder dass die Folie der Abschirmung (3) um den Behälter (1) gewickelt oder angeordnet ist.

3. Abgabevorrichtung nach Anspruch 1 oder 2, **dadurch gekennzeichnet, dass** die Leiter (32, 33, 34) schleifenfrei und/oder frei von geschlossenen Leiterschleifen ausgebildet sind.

4. Abgabevorrichtung nach einem der vorangehenden Ansprüche und/oder dass die Leiter (32, 33, 34) eine Dicke von höchstens 3 mm, insbesondere von zwischen 50 µm und 150 µm, aufweisen.

5. Abgabevorrichtung nach einem der vorangehenden Ansprüche, **dadurch gekennzeichnet, dass**
auf der Folie (31) ein Kommunikationscontroller (61) und/oder eine Anzahl von Kapazitätsmesseinrichtungen zur Ermittlung der Kapazität zwischen den Messelektroden (21, 22) und/oder eine Recheneinheit (6) und/oder eine Antenne (62) zur Übertragung von mit den Messelektroden (21, 22) ermittelten Messwerten oder daraus abgeleiteten Werten, insbesondere von Füllstandswerten (F), angeordnet sind, und dass diese auf der Folie (31) angeordneten Einheiten vorzugsweise in einem gemeinsamen Gehäuse eines Halbleiterchips integriert sind.

6. Abgabevorrichtung nach einem der vorangehenden Ansprüche, **dadurch gekennzeichnet, dass** die beiden gegenüberliegenden Messelektroden (21, 22) an eine Kapazitätsmesseinrichtung (41) angeschlossen sind, und dass vorzugsweise der von der Kapazitätsmesseinrichtung (41) ermittelte Kapazitätswert (C₁) einer Recheneinheit (6) zugeführt ist, die aufgrund des ermittelten Kapazitätswerts (C₁) mittels einer vorgegebenen abgespeicherten Kalibrierfunktion den Füllstand (F) der Flüssigkeit (12) im Behälter (1) bestimmt und an ihrem Ausgang zur Verfügung hält.

7. Abgabevorrichtung nach Anspruch 6, **dadurch gekennzeichnet, dass** einer der drei Leiter (32, 33, 34), insbesondere der als Kammleiter ausgebildete zweite Leiter (33), mit dem Massenanschluss der Kapazitätsmesseinrichtung (41) verbunden ist.

8. Abgabevorrichtung nach einem der vorangehenden Ansprüche, **gekennzeichnet durch** einen außerhalb oder im Bereich der Abschirmung (3) angeordneten, insbesondere kapazitiven, Berührungssensor (5), wobei vorzugsweise der Berührungssensor (5) den ersten Kammleiter (32) und den mäanderförmigen Leiter (34) der Abschirmung (3) als Sensorelektroden umfasst.

9. Abgabevorrichtung nach Anspruch 8, **dadurch gekennzeichnet, dass** die Sensorelektroden des Berührungssensors (5) an eine weitere Kapazitätsmesseinrichtung (44) angeschlossen sind, und dass vorzugsweise der von der weiteren Kapazitätsmesseinrichtung (44) ermittelte weitere Kapazitätswert (C') der Recheneinheit (6) zugeführt ist, und die Recheneinheit (6) für den Fall, dass der ermittelte weitere Kapazitätswert (C') einen vorgegebenen Schwellenwert (T) übersteigt, die Weiterleitung des von ihr ermittelten Füllstands (F) unterdrückt oder als ungültig kennzeichnet.

10. Abgabevorrichtung nach einem der vorangehenden Ansprüche, **dadurch gekennzeichnet, dass** der Behälter (1) ein Innenvolumen aufweist, das abgesehen vom Bereich der Öffnung (11) einen konstanten Innenquerschnitt aufweist,
wobei ein den Behälter (1) und die darin befindliche Flüssigkeit (12) abschließender und abdichtender Kolben (13) vorgesehen ist, dessen Außenquerschnitt dem Querschnitt des Innenvolumens des Behälters (1) entspricht und der im Inneren des Behälters (1) verschiebbar angeordnet ist, sodass bei Vorschub des Kolbens (13) zur Öffnung (11) hin die Flüssigkeit (12) durch die Öffnung (11) aus dem Behälter (1) abgegeben wird.

11. Abgabevorrichtung nach einem der vorangehenden Ansprüche, **dadurch gekennzeichnet, dass** am Behälter (1) eine Vielzahl von Paaren von zusätzlichen Messelektroden (23, 24; 25, 26) angeordnet sind, wobei insbesondere an jedes Paar von zusätzlichen Messelektroden (23, 24; 25, 26) jeweils eine zusätzliche dem Paar von zusätzlichen Messelektroden (23, 24; 25, 26) nachgeschaltete Kapazitätsmesseinrichtung (42, 43) vorgesehen ist, die den ermittelten Kapazitätswert (C₂, C₃) an die Recheneinheit (6) abgibt.

12. Abgabevorrichtung nach einem der vorangehenden Ansprüche, **dadurch gekennzeichnet, dass** die jeweils einander paarweise zugeordneten Messelektroden (21, 22; 23, 24; 25, 26) einander in Umfangsrichtung des Behälters (1), insbesondere diametral, gegenüberliegen und insbesondere in Richtung des Vorschubs des Kolbens (13) auf derselben Höhe liegen.

13. Abgabevorrichtung nach Anspruch 12, **dadurch gekennzeichnet, dass** jeweils benachbarte Paare von Messelektroden (21, 22; 23, 24; 25, 26) in Richtung des Vorschubs des Kolbens (13) beabstandet angeordnet sind und/oder dass die Breite der Messelektroden (21, 22; 23, 24; 25, 26) in Richtung (V) des Vorschubs des Kolbens (13) der Breite des Kolbens (13) in dessen Vorschubrichtung (V) entspricht.

14. Abgabevorrichtung nach einem der vorangehenden Ansprüche, **dadurch gekennzeichnet,**
- **dass** die Messelektroden (21, 22; 23, 24; 25, 26) flächenhaft auf der Außenoberfläche des Behälters (1) angeordnet sind und insbesondere die Form eines Rechtecks, eines Dreiecks, eines Trapezes oder eines Parallelogramms aufweisen, und/oder
- **dass** je zwei der einander paarweise zugeordneten Messelektroden (21, 22; 23, 24; 25, 26) durch zwei ineinandergreifende Kammleiter ausgebildet sind, die im Außenbereich, insbesondere an der Außenwand, des Behälters (1) angeordnet sind.

15. Abgabevorrichtung nach einem der vorangehenden Ansprüche, **dadurch gekennzeichnet dass** außerhalb des Behälters (1) zwischen dem Behälter (1) und der Abschirmung (3) ein Träger angeordnet ist, auf dem die Messelektroden (21, 22; 23, 24; 25, 26) angeordnet sind, wobei der Träger vorzugsweise am Behälter (1) anliegt und/oder dass die Messelektroden (21, 22; 23, 24; 25, 26) auf der am Behälter (1) anliegenden Wand des Trägers angeordnet sind, wobei insbesondere ein Teil des Gehäuses der Abgabevorrichtung (100) als Träger ausgebildet ist oder der Träger mit dem Gehäuse verbunden ist.

16. Abgabevorrichtung nach einem der vorangehenden Ansprüche, **dadurch gekennzeichnet, dass** an die Recheneinheit (6) ein Kommunikationscontroller (61) mit einer ihm nachgeschalteten Antenne (62) angeschlossen ist,
wobei insbesondere die Antenne (62) im Außenbereich der Abschirmung (3) oder unmittelbar auf der Abschirmung (3), mit der Abschirmung (3) jedoch nicht elektrisch leitfähig verbunden, angeordnet ist.

17. Verfahren zur Bestimmung und Validierung des Füllstands (F) in einem Behälter (1), der insbesondere in einer Abgabevorrichtung (100) nach einem der vorangehenden Ansprüche angeordnet ist, wobei zumindest ein Paar von im Außenbereich des Behälters (1) einander gegenüberliegend angeordneten, insbesondere mit einer äußeren Abschirmung (3) versehenen, Messelektroden (21, 22) zur Kapazitätsmessung vorgesehen ist,
wobei die Kapazität (C₁) zwischen den beiden Messelektroden (21, 22) ermittelt wird und aufgrund der ermittelten Kapazität (C₁) gemäß einer vorgegebenen Kalibrierfunktion ein Füllstandswert (F) bestimmt wird,
**dadurch gekennzeichnet,**
- **dass** eine weitere Kapazität (C') mit im Außenbereich der Messelektroden (21, 22) im Bereich der Abschirmung (3), insbesondere auf der Abschirmung (3), angeordneten Leitern (33, 34) ermittelt wird,
- **dass** die weitere Kapazität (C') mit einem Schwellenwert (T) verglichen wird, und
- **dass** der Füllstandswert (F) nur dann als gültig angesehen wird, wenn die weitere Kapazität (C') unterhalb des Schwellenwerts (T) liegt.

18. Verfahren nach Anspruch 17, **dadurch gekennzeichnet, dass** der Füllstandwert (F) und/oder eine Aussage über die Gültigkeit des Füllstandswertes (F) durch codierte elektromagnetischer Datenübertragung, insbesondere durch Lastmodulation, an ein externes Datenkommunikationsgerät übertragen wird.

19. Verfahren nach Anspruch 17 oder 18, **dadurch gekennzeichnet, dass** jeweils die Kapazitäten (C₁, C₂, C₃) einer Vielzahl von, insbesondere drei, Paaren von Messelektroden (21-26), die einander im Außenbereich des Behälters (1) gegenüberliegen, ermittelt werden und der Füllstandswert (F) aufgrund der Kapazitäten ermittelt wird.

20. Verfahren nach einem der Anspruch 19, **dadurch gekennzeichnet, dass**
a) für eine Anzahl von Füllständen (F) jeweils ein Referenzvektor (V_{ref}) umfassend die Kapazitäten (C₁, C₂, C₃) zwischen den einzelnen Paaren von Messelektroden (21-26) als Komponenten zur Verfügung gestellt werden, und
b) jedem dieser Vektoren der jeweilige Füllstand (F) zugeordnet wird,
c) ein Vektor (Vₘₑₛₛ) umfassend die einzelnen ermittelten Kapazitäten (C₁, C₂, C₃) ermittelt wird,
d) eine Anzahl von Referenzvektoren (V_{ref}) gesucht wird, die vom Vektor (Vₘₑₛₛ) den geringsten, insbesondere euklidischen, Abstand aufweisen,
e) eine Interpolationsfunktion gebildet wird, die bei Anwendung auf die in Schritt b) aufgefundenen Referenzvektoren (V_{ref}) den jeweiligen, diesen Referenzvektoren (V_{ref}) zugeordneten Füllstand (F) ergibt,
f) die Interpolationsfunktion auf den Vektor (Vₘₑₛₛ) angewendet wird und das Ergebnis als Füllstand herangezogen wird.

## Claims

1. An administration device for administering liquids (12), in particular liquid drugs, to persons, the device comprising:
- a container (1) filled with the liquid (12) and having an opening (11) formed therein on one end to administer the liquid (12),
- at least one pair of capacitive measurement electrodes (21, 22) disposed in the outer area, in particular on the wall, of the container (1) opposite one another, to determine the permittivity of the medium in the space between the measurement electrodes; and
- a cover (3) surrounding the measurement electrodes (21, 22) in a jacket-like manner and disposed around the container (1),
wherein the cover (3) has a film (31) coated with conductors (32, 33, 34) in a form of conductive tracks,
**characterised in that**
three separate conductors (32, 33, 34) are formed on the film (31), wherein the first and second conductors (32, 33) are formed as mutually engaging comb conductors, and the third conductor (34) is formed in a meander-shaped manner between the two comb conductors (32, 33).

2. The administration device according to claim 1, **characterised**
**in that** the area between the liquid (12) and the measurement electrodes (21, 22) is free of the cover (3), and/or
**in that** the cover (3) is spaced apart from the measurement electrodes (21, 22), and/or in that the film of the cover (3) is wound or arranged around the container (1).

3. The administration device according to claim 1 or 2, **characterised in that** the conductors (32, 33, 34) are formed in a loop-free manner and/or free of closed conductor loops.

4. The administration device according to any one of the preceding claims and/or in that the conductors (32, 33, 34) have a thickness of at most 3 mm, in particular of between 50 µm and 150 µm.

5. The administration device according to any one of the preceding claims, **characterised in that**
a communications controller (61) and/or a number of capacitance measuring devices for establishing the capacitance between the measurement electrodes (21, 22) and/or a processing unit (6) and/or an antenna (62) for transmitting measurement values established by the measurement electrodes (21, 22) or values derived therefrom, in particular fill level values (F), are arranged on the film (31), and **in that** these units arranged on the film (31) are preferably integrated in a common housing of a semiconductor chip.

6. The administration device according to any one of the preceding claims, **characterised in that** the two measurement electrodes (21, 22) disposed opposite one another are connected to a capacitance measuring device (41), and **in that** the capacitance value (C₁) established by the capacitance measuring device (41) is preferably supplied to a processing unit (6), which, based on the established capacitance value (C₁), determines the fill level (F) of the liquid (12) in the container (1) by means of a specified stored calibration function and makes the determined fill level available at an output.

7. The administration device according to claim 6, **characterised in that** one of the three conductors (32, 33, 34), in particular the second conductor (33) formed as a comb conductor, is connected to the ground connection of the capacitance measuring device (41).

8. The administration device according to any one of the preceding claims, **characterised by** a contact sensor (5), in particular a capacitive contact sensor, disposed outside or in the area of the cover (3), wherein the contact sensor (5) preferably comprises the first comb conductor (32) and the meander-shaped conductor (34) of the cover (3) as sensor electrodes.

9. The administration device according to claim 8, **characterised in that** the sensor electrodes of the contact sensor (5) are connected to a further capacitance measuring device (44), and **in that** the further capacitance value (C') established by the further capacitance measuring device (44) is fed to the computing unit (6), and the computing unit (6) suppresses the forwarding of the fill level (F) established by the processing unit or marks the fill level invalid if the established further capacitance value (C') exceeds a specified threshold value (T).

10. The administration device according to any one of the preceding claims, **characterised in that** the container (1) has an internal volume having a constant internal cross section with the exception of the area of the opening (11),
wherein a plunger (13) sealing the container (1) and the liquid (12) contained therein is provided, the outer cross section of the plunger corresponding to the cross section of the internal volume of the container (1), and the plunger being disposed movably inside the container (1), such that, when the plunger (13) is moved towards the opening (11), the liquid (12) is administered out of the container (1) through the opening (11).

11. The administration device according to any one of the preceding claims, **characterised in that** a plurality of pairs of additional measurement electrodes (23, 24; 25, 26) are disposed on the container (1), wherein additional capacitance measuring devices (42, 43) downstream of the pairs of additional measurement electrodes (23, 24; 25, 26) are provided, in particular one on each pair of additional measurement electrodes (23, 24; 25, 26), and output the established capacitance value (C₂, C₃) to the processing unit (6).

12. The administration device according to any one of the preceding claims, **characterised in that** the measurement electrodes (21, 22; 23, 24; 25, 26) associated with one another in pairs are mutually opposed, in particular diametrically, in the peripheral direction of the container (1), and in particular are arranged at the same height in particular in the direction of movement of the plunger (13).

13. The administration device according to claim 12, **characterised in that** adjacent pairs of measurement electrodes (21, 22; 23, 24; 25, 26) are disposed at a distance in a direction of movement of the plunger (13), and/or
**in that** the width of the measurement electrodes (21, 22; 23, 24; 25, 26) in the direction (V) of movement of the plunger (13) corresponds to the width of the plunger (13) in the direction of movement (V) thereof.

14. The administration device according to any one of the preceding claims, **characterised**
- **in that** the measurement electrodes (21, 22; 23, 24; 25, 26) are arranged two-dimensionally on the outer surface of the container (1) and in particular have the shape of a rectangle, a triangle, a trapezium or a parallelogram, and/or
- **in that** each two of the measurement electrodes (21, 22; 23, 24; 25, 26) associated with one another in pairs are formed by two mutually engaging comb conductors that are disposed in the outer area, in particular on the outer wall, of the container (1).

15. The administration device according to any one of the preceding claims, **characterised in that** a support is disposed outside the container (1), between the container (1) and the cover (3), the measurement electrodes (21, 22; 23, 24; 25, 26) being disposed on the support, wherein the support preferably abuts the container (1), and/or **in that** the measurement electrodes (21, 22; 23, 24; 25, 26) are disposed on the wall of the support abutting the container (1), wherein in particular a part of the housing of the administration device (100) is formed as a support or the support is connected to the housing.

16. The administration device according to any one of the preceding claims, **characterised in that** a communications controller (61) with a downstream antenna (62) is connected to the processing unit (6),
wherein in particular the antenna (62) is disposed in the outer area of the cover (3) or directly on the cover (3), but is not connected to the cover (3) in an electrically conductive manner.

17. A method for determining and validating the fill level (F) in a container (1) disposed in particular in an administration device (100) according to any one of the preceding claims, wherein at least one pair of measurement electrodes (21, 22) for measuring a capacitance, disposed opposite one another in the outer area of the container (1) and in particular provided with an outer cover (3), is provided, wherein the capacitance (C₁) between the two measurement electrodes (21, 22) is established and a fill level value (F) is determined based on the established capacitance (C₁) according to a specified calibration function,
**characterised in that**
- a further capacitance (C') is established using conductors (33, 34) arranged in the outer area of the measurement electrodes (21, 22) in the area of the cover (3), in particular on the cover (3),
- **in that** the further capacitance (C') is compared with a threshold value (T), and
- **in that** the fill level (F) is only considered valid if the further capacitance (C') is below the threshold value (T).

18. The method according to claim 17, **characterised in that** the fill level value (F) and/or information regarding the validity of the fill level value (F) is transmitted to an external data communications device by encoded electromagnetic data transmission, in particular by load modulation.

19. The method according to claim 17 or 18, **characterised in that** the capacitances (C₁, C₂, C₃) of a plurality of pairs, in particular three pairs, of measurement electrodes (21-26), which are disposed opposite one another in the outer area of the container (1) are established, and the fill level value (F) is established on the basis of the capacitances.

20. The method according to claim 19, **characterised in that**
a) a reference vector (V_{ref}) containing the capacitances (C₁ C₂, C₃) between the individual pairs of measurement electrode (21-26) is made available as component for each of a number of fill levels (F), and
b) a fill level (F) is associated with each of these vectors,
c) a vector (Vₘₑₛₛ) containing the individual established capacitances (C₁, C₂, C₃) is established,
d) a number of reference vectors (V_{ref}) that are at the smallest, in particular Euclidean distance from the vector (Vₘₑₛₛ) is sought,
e) an interpolation function is formed that, when applied to the reference vectors (V_{ref}) found in step b), provides the fill levels (F) associated with these reference vectors (V_{ref}), and
f) the interpolation function is applied to the vector (Vₘₑₛₛ) and the result is used as the fill level.

## Revendications

1. Dispositif d'administration servant à administrer des liquides (12), notamment des médicaments liquides, à des personnes, comprenant
- un contenant (1) rempli du liquide (12) et présentant à une extrémité un orifice (11) servant à administrer le liquide (12),
- au moins une paire d'électrodes de mesure capacitives (21, 22) agencées en vis-à-vis dans la zone externe, en particulier sur la paroi, du contenant (1), pour déterminer la permittivité du milieu respectif situé dans l'espace intermédiaire entre les électrodes de mesure et
- une protection (3) située autour du contenant (1) étant agencée pour entourer les électrodes de mesure (21, 22) à la manière d'une enveloppe
dans lequel la protection (3) est conçue comme une feuille (31) revêtue de conducteurs (32, 33, 34) sous la forme de pistes conductrices
**caractérisé en ce que**
trois conducteurs séparés (32, 33, 34) sont conçus sur la feuille (31), dans lequel le premier et le deuxième conducteur (32, 33) sont conçus comme des conducteurs en peigne s'engrenant l'un dans l'autre et le troisième conducteur (34) est conçu en forme de méandres et placé entre les deux conducteurs en peigne (32, 33).

2. Dispositif d'administration selon la revendication 1, **caractérisé en ce**
**que** la zone entre le liquide (12) et les électrodes de mesure (21, 22) ne contient pas la protection (3) et/ou
en ce que la protection (3) est à distance des électrodes de mesure (21, 22) et/ou en ce que la feuille de la protection (3) est enroulée ou disposée autour du contenant (1).

3. Dispositif d'administration selon les revendications 1 ou 2, **caractérisé en ce que** les conducteurs (32, 33, 34) sont conçus sans boucle et/ou dépourvus de boucles conductrices fermées.

4. Dispositif d'administration selon une des revendications précédentes et/ou en ce que les conducteurs (32, 33, 34) présentent une épaisseur maximum de 3 mm, notamment entre 50 µm et 150 µm.

5. Dispositif d'administration selon une des revendications précédentes, caractérisé en ce
sur la feuille (31), sont agencés un organe de commande de communication (61) et/ou un certain nombre de dispositifs de mesure de capacité servant à déterminer la capacité entre les électrodes de mesure (21, 22) et/ou une unité arithmétique (6) et/ou une antenne (62) servant à la transmission de valeurs de mesures déterminées avec les électrodes de mesure (21, 22) ou de valeurs qui en découlent, notamment de valeurs d'état de remplissage (F) et en ce que ces unités disposées sur la feuille (31) sont de préférence intégrées dans un boîtier commun d'une puce à semi-conducteur.

6. Dispositif d'administration selon une des revendications précédentes, **caractérisé en ce que** les deux électrodes de mesure en vis-à-vis (21, 22) sont raccordées à un dispositif de mesure de capacité (41) et **en ce que**, de préférence, la valeur de capacité (C₁) déterminée par le dispositif de mesure de capacité (41) est transférée à une unité arithmétique (6), qui, sur la base de la valeur de capacité (C₁) déterminée, détermine, au moyen d'une fonction d'étalonnage mémorisée prédéfinie, l'état de remplissage (F) du liquide (12) dans le contenant (1) et le tient à disposition au niveau de sa sortie.

7. Dispositif d'administration selon la revendication 6, **caractérisé en ce qu'**un des trois conducteurs (32, 33, 34), notamment le deuxième conducteur (33) conçu sous forme de conducteur en peigne, est raccordé au raccordement à la masse du dispositif de mesure de capacité (41).

8. Dispositif d'administration selon une des revendications précédentes, **caractérisé par** un capteur de contact (5), notamment capacitif, disposé en dehors ou dans la zone de la protection (3), dans lequel, de préférence, le capteur de contact (5) comprend le premier conducteur en peigne (32) et le conducteur (34) en forme de méandres de la protection (3) comme électrodes de capteur.

9. Dispositif d'administration selon la revendication 8, **caractérisé en ce que** les électrodes de capteur du capteur de contact (5) sont raccordées à un autre dispositif de mesure de capacité (44) et **en ce que**, de préférence, l'autre valeur de capacité (C') déterminée par l'autre dispositif de mesure de capacité (44) est transférée à l'unité arithmétique (6) et l'unité arithmétique (6), au cas où l'autre valeur de capacité (C') déterminée dépasse une valeur seuil prédéfinie (T), supprime ou considère invalide la transmission de l'état de remplissage (F) qu'elle a déterminée.

10. Dispositif d'administration selon une des revendications précédentes, **caractérisé en ce que** le contenant (1) présente un volume interne, qui présente, outre la zone de l'ouverture (11), une section transversale interne constante,
dans lequel un piston (13) fermant et scellant le contenant (1) et le liquide contenu à l'intérieur (12) est prévu, dont la section transversale externe correspond à la section transversale du volume interne du contenant (1) et qui est disposé à l'intérieur du contenant (1) de manière coulissante, de sorte que lors de l'avancée du piston (13) vers l'ouverture (11), le liquide (12) soit administré à travers l'ouverture (11) hors du contenant (1).

11. Dispositif d'administration selon une des revendications précédentes, **caractérisé en ce qu'**une pluralité de paires d'électrodes de mesure supplémentaires (23, 24 ; 25, 26) est disposée sur le contenant (1), dans lequel, notamment, pour chaque paire d'électrodes de mesure supplémentaires (23, 24 ; 25, 26), respectivement, un dispositif de mesure de capacité (42, 43) supplémentaire est prévu en aval de la paire d'électrodes de mesure supplémentaires (23, 24 ; 25, 26), qui émet les valeurs de capacité (C₂, C₃) déterminées vers l'unité arithmétique (6).

12. Dispositif d'administration selon une des revendications précédentes, **caractérisé en ce que** les électrodes de mesure (21, 22 ; 23, 24 ; 25, 26) disposées respectivement par paire sont disposées opposées les unes aux autres dans la direction circonférentielle du contenant (1), notamment diamétralement opposées et, en particulier, dans la direction d'avance du piston (13) à la même hauteur.

13. Dispositif d'administration selon la revendication 12, **caractérisé en ce que** des paires respectivement voisines d'électrodes de mesure (21, 22 ; 23, 24 ; 25, 26) sont disposées espacées dans la direction d'avance du piston (13) et/ou
**en ce que** la largeur des électrodes de mesure (21, 22 ; 23, 24 ; 25, 26) dans la direction (V) d'avance du piston (13) correspond à la largeur du piston (13) dans sa direction d'avance (V).

14. Dispositif d'administration selon une des revendications précédentes, **caractérisé en ce**
- **que** les électrodes de mesure (21, 22 ; 23, 24 ; 25, 26) sont disposées de manière plane sur la surface externe du contenant (1) et présentent notamment la forme d'un rectangle, d'un triangle, d'un trapèze ou d'un parallélogramme et/ou
- en ce que respectivement deux des électrodes de mesure (21, 22 ; 23, 24 ; 25, 26) disposées par paires sont conçus par deux conducteurs en peigne s'engrenant, qui sont disposés dans la zone externe, notamment sur la paroi externe, du contenant (1).

15. Dispositif d'administration selon une des revendications précédentes, **caractérisé en ce que**, en dehors du contenant (1) entre le contenant (1) et la protection (3), un support est disposé, sur lequel les électrodes de mesure (21, 22 ; 23, 24 ; 25, 26) sont disposées, dans lequel le support repose de préférence sur le contenant (1) et/ou **en ce que** les électrodes de mesure (21, 22 ; 23, 24 ; 25, 26) sont disposées sur la paroi du support reposant sur le contenant (1), dans lequel, notamment, une partie du boîtier du dispositif d'administration (100) est conçue comme un support ou le support est raccordé au boîtier.

16. Dispositif d'administration selon une des revendications précédentes, **caractérisé en ce qu'**un organe de commande de communication (61) avec une antenne (62) raccordée à celui-ci en aval, est raccordé à l'unité arithmétique (6),
dans lequel, notamment, l'antenne (62) est disposée dans la zone externe de la protection (3) ou directement sur la protection (3), mais n'est cependant pas reliée de manière électroconductrice à la protection (3).

17. Procédé de détermination et de validation de l'état de remplissage (F) dans un contenant (1), qui est notamment disposé dans un dispositif d'administration (100) selon une des revendications précédentes, dans lequel au moins une paire d'électrodes de mesure (21, 22) servant à mesurer la capacité, disposées en vis-à-vis, dotées notamment d'une protection externe (3) est prévue dans la zone externe du contenant (1),
dans lequel la capacité (C₁) entre les deux électrodes de mesure (21, 22) est déterminée et, sur la base de la capacité (C₁) déterminée, une valeur d'état de remplissage (F) est déterminée conformément à une fonction d'étalonnage prédéfinie, **caractérisé en ce**
- **qu'**une autre capacité (C') est déterminée, avec des conducteurs (33, 34) disposés dans la zone externe des électrodes de mesure (21, 22) dans la zone de la protection (3), notamment sur la protection (3),
- en ce que l'autre capacité (C') est comparée à une valeur seuil (T) et
- en ce que la valeur d'état de remplissage (F) est uniquement considérée comme valide lorsque l'autre capacité (C') est inférieure à la valeur seuil (T).

18. Procédé selon la revendication 17, **caractérisé en ce que** la valeur d'état de remplissage (F) et/ou une affirmation de la validité de la valeur d'état de remplissage (F) est transmise par transmission de données électromagnétique codée, notamment par modulation de charge, à un appareil de communication de données externe.

19. Procédé selon les revendications 17 ou 18, **caractérisé en ce que** les capacités (C₁, C₂, C₃) d'une pluralité de, en particulier trois, paires d'électrodes de mesure (21-26), situées en vis-à-vis dans la zone externe du contenant (1), sont déterminées et la valeur d'état de remplissage (F) est déterminée sur la base des capacités.

20. Procédé selon la revendication 19, **caractérisé en ce**
a) **que**, pour un certain nombre d'états de remplissage (F), respectivement, un vecteur de référence (V_{ref}) comprenant les capacités (C₁ C₂, C₃) entre les paires individuelles des électrodes de mesure (21-26) est placé à disposition en guise de composants et
b) chacun de ces vecteurs est attribué à l'état de remplissage respectif (F),
c) un vecteur (Vₘₑₛₛ) comprenant les capacités (C₁, C₂, C₃) individuelles déterminées est déterminé,
d) un certain nombre de vecteurs de référence (V_{ref}) est recherché, qui présentent la distance la plus faible, notamment euclidienne, par rapport au vecteur (Vₘₑₛₛ),
e) une fonction d'interpolation est conçue, qui, lors de l'utilisation sur les vecteurs de référence (V_{ref}) trouvés dans l'étape b), fournit l'état de remplissage (F) respectif attribué à ces vecteurs de référence (V_{ref}),
f) la fonction d'interpolation est utilisée sur le vecteur (Vₘₑₛₛ) et le résultat est déduit en guise d'état de remplissage.
